**Europäisches Patentamt**

**European Patent Office**

**Office européen des brevets**

(19)

(11) Veröffentlichungsnummer: **0 242 351 B1**

# EUROPÄISCHE PATENTSCHRIFT

(12)

(45) Veröffentlichungstag der Patentschrift: **03.07.91**

(51) Int. Cl.⁵: **A61M 5/14**

(21) Anmeldenummer: **87890065.3**

(22) Anmeldetag: **03.04.87**

(54) **Vorrichtung zur strömungskonstanten Abgabe flüssiger Arzneimittel.**

(30) Priorität: **04.04.86 AT 891/86**

(43) Veröffentlichungstag der Anmeldung:
**21.10.87 Patentblatt 87/43**

(45) Bekanntmachung des Hinweises auf die
Patenterteilung:
**03.07.91 Patentblatt 91/27**

(84) Benannte Vertragsstaaten:
**BE CH DE ES FR GB GR IT LI LU NL SE**

(56) Entgegenhaltungen:
**EP-A- 0 128 703**
**WO-A-83/02063**
**US-A- 4 201 207**
**US-A- 4 274 407**
**US-A- 4 419 096**

(73) Patentinhaber: **Thoma, Herwig, Prof. Dipl.-Ing.
Dr.
Wiesengasse 3
A-1140 Wien(AT)**

(72) Erfinder: **Thoma, Herwig, Univ. Prof. Dipl.-Ing.
Dr.
Wiesengasse 3
A-1140 Wien(AT)**
Erfinder: **Krötlinger, Michael, Dr.
Ebnerstrasse 30
A-3160 Traisen Niederösterreich(AT)**

(74) Vertreter: **Krause, Ernst, Dipl.-Ing. et al
Dipl.- Ing. Krause, Ernst Dipl. Ing. Casati,
Wilhelm Patentanwälte Amerlingstrasse 8
A-1061 Wien(AT)**

**Beschreibung**

Die Erfindung bezieht sich auf eine Vorrichtung zur strömungskonstanten Abgabe flüssiger Arzneimittel, bestehend aus einem Gehäuse mit einem Unterteil, einem im Gehäuse untergebrachten, komprimierbaren flexiblen Reservoir zur Aufnahme des Arzneimittels, gegebenenfalls einem Füllventil zur Füllung des Reservoirs, einem mit dem Reservoir verbundenen bzw. verbindbaren Ausströmkanal definierten Querschnitts und definierter Länge, sowie einer zur Applikation geeigneten Ausgangskanüle, wobei mit dem Gehäuse-Unterteil ein zylinderförmiger Grundteil verbunden ist, um welchen Grundteil herum das flexible Reservoir für das Arzneimittel angeordnet ist. Solch eine Vorrichtung ist aus der US-A-4419096 bekannt. Bei der bekannten Einrichtung ist der Grundteil als Birne ausgebildet und in das Reservoir in der Art eines Kernes eingesetzt, um die beim Entleeren des Reservoirs sich einstellende Birnenform des Reservoirs bis zum Ende der Entleerung aufrechtzuerhalten, um eine Druckspitze, die sich sonst gegen Entleerungsende einstellen würde, nicht entstehen zu lassen.

Für das Reservoir ist das Füllventil dann vorzusehen, wenn die Vorrichtung nicht als Wegwerfeinheit, d.h. zur nur einmaligen Verwendung bestimmt ist. Als Wegwerfeinheit kann die Vorrichtung mit bereits gefülltem Reservoir zur Verfügung gestellt werden.

Infusionspumpen werden am Menschen heute nur in seltenen Fällen bei bestimmten Krankheiten verwendet. In der Anwendung gibt es grundsätzlich zwei Gebiete.

Beim medikamentösen Einsatz kann mit Hilfe einer pharmakologischen Substanz ein körperliches Leiden (z.B.Schmerz) oder eine Körperfunktion (z.B.Blutgerinnung) verbessert oder ein akuter Krisenzustand behoben werden. Ein Beispiel für letztere Anwendung bezieht sich auf die Infusion antiarrythmischer Pharmaka bei herzkranken Patienten, die nicht über den sogenannten Herzschrittmacher betreut werden können. Solche Vorrichtungen dienen dazu, einen gefährlichen Herzanfall zu vermeiden, wobei der Zeitpunkt der Infusion beispielsweise von der Arrhytmie der Herzaktion ausgelöst wird. Diese Pumpen sind üblicherweise implantiert. Eine andere Gruppe von Vorrichtungen dient dem Ersatz von Körperflüssigkeiten jener Organe, die krankheitsbedingt geschädigt wurden. Der Ersatz solcher Flüssigkeiten fällt in den Bereich der Endokrinologie. Bekanntestes Beispiel für den Ausfall eines solchen Organes ist die Bauchspeicheldrüse (Pankreas) , welche unter anderem das lebensnotwendige Insulin produziert. Heute werden solche Patienten hauptsächlich durch Spritzen von Depotinsulin versorgt, wobei jedoch nach einigen Jahren Organschäden zu bemerken sind. Eine weitere Gruppe betrifft die Zufuhr mangelnder flüssiger Substanzen (Blutersatz .... künstliche Ernährung).

In jedem Fall werden entweder ein Arzneimittel bzw. die für den Körper notwendigen Substanzen oder die notwendigen Körperflüssigkeiten in einem Speicher deponiert, von dort über eine Pumpvorrichtung in den Körper gebracht (appliziert), wobei der Speicherinhalt in bestimmten Intervallen nachgefüllt werden muß. Die Applikation kann oral (Resorption der Flüssigkeit über den Magen-Darm-Trakt), subkutan (Resorption über das Lymphsystem) oder venös bzw. arteriell erfolgen. Intraperitoneal wird heute nur Insulin appliziert.

Die vorliegende Erfindung kann insbesondere zur subkutanen Substitution von Insulin verwendet werden. Im Rahmen heute möglicher Formen der Therapie kann eine Pumpe mit strömungskonstanter Abgabe ("Konstantratenpumpe") wie folgt eingesetzt werden:

1. Zur Abdeckung des basalen Anteiles, insbesondere dann, wenn, was häufig der Fall ist, "verzögertes Insulin" (Langzeitinsulin) schlecht wirkt,

2. bei peripherer Insulinresistenz, wobei sich durch die gleichmäßige, über den Tag verteilte Applikation von Normalinsulin in Vergleich zu Langzeitinsulin ein positiv therapeutischer Effekt ergibt,

3. bei Erkrankung nach dem sogenannten Typ II als Schwerpunkt der Anwendung, da solche Patienten nach Abdekkung des basalen Anteiles über die erfindungsgemäße Vorrichtung mit Hilfe der noch vorhandenen Restfunktion des Pankreas (Bauchspeicheldrüse), den prandialen (Essen)-Anteil abdecken können und darüber hinaus, aufgrund der vorhandenen Sensorfunktion, der prandiale Ersatz vom Körper reguliert werden kann.

Der Einsatz der vorliegenden Erfindung ist jedoch keineswegs auf die subkutane Applikation von Insulin begrenzt. Vielmehr ist diese einfache und billige Form der "Langzeitinjektion" für viele notwendige Einsätze optimal; nämlich etwa

* zur exakt dosierten Applikation von Medikamenten zur Blutgerinnung,

* zur u.U. auch lokalen Behandlung mit Antibiotika und

* zur lokalen Schmerztherapie.

Auch läßt sich die erfindungsgemäße Vorrichtung implantieren und lassen sich im Vergleich zu heute üblichen Pumpen (Infusaid) eine Reihe von Vorteilen definieren.

Die nachfolgenden Erläuterungen von Lösungsvorschlägen für die verschiedenen Probleme an Hand bestehender Vorrichtungen bezieht sich schwerpunktsmäßig auf extern am Körper angebrachte und bzw. oder implantierbare Insulinpumpen mit konstanter Strömung. Übliche Infusionssysteme, welche beispielsweise am Bett des Patienten angebracht sind, sind nicht Gegenstand der weiteren Erläuterungen.

Bei der Entwicklung von Infusionspumpen können aus biologisch-technischer Sicht folgende Problemkreise definiert werden:

1. Dauerhaltbarkeit der Pumpe,

2. Ausfallsicherheit und Redundanz,

3. Steuerung (Programm) sowie Antrieb der Pumpe,

4. Biologischer Sensor zur automatischen Steuerung (closed loop) der Infusion,

5. Haltbarkeit und Funktion der Ausgangskanüle,

6. Energieversorgung und

7. Sicherheit.

Weitere Kriterien betreffen Merkmale wie Implantierbarkeit und Preis.

In der Praxis haben sich zur Implantation heute nur Pumpen mit konstanter Pumprate bewährt: Ein unter Überdruck stehender flexibler Insulinbehälter liefert über eine Strömungsdrossel oder eine Reduktionskanüle eine druckproportionale Menge von Insulin. Der Druck wird bei solchen Systemen meist mit Hilfe eines Zwischenmembrans über Treibgas (FREON) erzeugt. Treibgas, letztlich als Energiequelle der Pumpe, wird auch im Rahmen modifizierter Entwicklungen verwendet. So ist in der EP-OS 0128703 zur Dosierung der Flüssigkeit ein vom Patienten extern zu bedienendes Ventil beschrieben. Eine interessante Idee - mit allen Problemen hohen Energieverbrauchs behaftet - findet sich in der US-PS 4505710. Die Steuerung eines Kolbens erfolgt dort über zwei mit Treibgas vorgespannte, reziproke Kammern, wobei zur Verschiebung des Kolbens jeweils eine Seite elektrisch aufgeheizt wird und durch den höheren Dampfdruck eine Verschiebung des Kolbens erreicht wird. Demgemäß fällt dort die Strömungsdrossel weg. Dies gilt auch für den Gegenstand, welcher in der EP-OS 0091624 beschrieben ist. Auch dabei wird als Energiequelle Dampfdruck verwendet. Der Dampf passiert jedoch ein semipermeables Membran. Der Durchtritt des Gases erfolgt demgemäß zeitlich verzögert. Ebenfalls mit Überdruck wird die Pumpe gemäß der EP-OS 0098893 betrieben. Bei dieser Konstruktion befindet sich zwischen einer Hochdruckgaskammer und dem Medikamentenbehälter ein weiterer geschlossener Raum. Ein Feinregulierventil des Gasbehälters versorgt den Zwischenraum mit Druck, der auf den flexiblen Medikamentenbehälter wirkt. Andere bekannte Pumpsysteme (DE-AS 2124062, US-PS 38356) beziehen sich auf die Weiterentwicklung von ursprünglich tragbaren Systemen. So hat beispielsweise die Firma Siemens eine miniaturisierte Rollen-Quetsch-Pumpe entwickelt, welche extern getragen werden kann und vom Patienten gesteuert wird. Auch eine implantierbare Steuerung wurde dafür entwickelt (DE-

OS 3018833, EP-A1 0019814). So nützlich eine solche Vorrichtung von der Funktion her ist - damit versorgte Patienten können theoretisch beliebig essen - ist deren Implantation mit großen Nachteilen verbunden, weil beispielsweise die Menge der infundierten Flüssigkeit von der Elastizität eines Silikonkautschukschlauches abhängt und bekanntermaßen Silikonkautschuk im Körper degeneriert. Die Lebensdauer solcher Pumpen beträgt daher nur etwa ein bis maximal zwei Jahre. Der Antrieb der Pumpen erfolgt über kleine Schrittmotoren mit Getriebe, deren Haltbarkeit insbesondere im biologischen Milieu keineswegs unbegrenzt ist.

Bei einer Einrichtung gemäß der WO-OS 8502344 wird ein Flüssigkeitsbehälter in Gestalt eines Metallfaltenbalges von einer Vorrichtung komprimiert, die im Prinzip aus einem elektromotorisch betriebenen Spindelantrieb besteht.

Auch osmotische Kräfte können zur Kompression des elastischen Medikamentenbehälters verwendet werden. So beschreibt beispielsweise die DE-AS 2201533 verschiedene Ausführungsformen jeweils zweier flexibler Behälter, wobei der erste (Medikamentenbehälter) vom zweiten osmotisch aktiven Behälter komprimiert wird. Gemeinsames Kennzeichen dieser Konstruktionen ist ein starres Außengehäuse, sodaß der durch Wasseraufnahme (Überangebot an osmotisch aktivem Material) sich vergrößernde osmotische Behälter seinen Druck auf den Medikamentenbehälter ausüben kann. Ein solches physikalisches Prinzip ist jedoch von dem der Erfindung zugrundeliegenden Prinzip verschieden. Eine Verbesserung des osmotischen Prinzips ist in der US-PS 3604417 beschrieben. Dieses allerdings aufwendige Prinzip trennt den Medikamententeil vom osmotischen Antrieb, enthält zwei verschiebbare Kolben sowie einen definierten Aktionsbereich für das osmotische Material.

Ein weiteres bekanntes Pumpsystem arbeitet über Membranpumpen mit vor- und nachgeschalteten Ventilen. Solche Pumpen sind zwar, was die Haltbarkeit betrifft, relativ gut zu dimensionieren, die Probleme, verursacht von den vom Flüssigkeitsstrom gesteuerten Ventilen (Ventilsitz, Dichtheit des Ventils, Rückzugsfeder....) sind jedoch extrem groß. Der wesentliche Nachteil eines solchen Ventils liegt jedoch in seiner Öffnungs- bzw. Schließcharakteristik. Geht man davon aus, daß pro Pumpzyklus etwa 1 - 2 $\mu$l gepumpt werden sollte, ist leicht einzusehen, daß allein für das Schließen des Ventils einige weitere $\mu$l benötigt werden. Tatsächlich findet man auch bei diversen Konstruktionen (US-PS 4265241 sowie US-PS 4360019) den Nachteil, daß ein Vielfaches des tatsächlich benötigten Volumens gepumpt wird, wobei die Genauigkeit der Pumpe von vielen Faktoren abhängt und eine Abweichung von 50 % (1 $\mu$l!) häufig auftritt. Bei der Konstruktion gemäß der US-PS 43 60 019

ist dem Aspekt der Sicherheit insofern Rechnung getragen, als manche Ventile redundant ausgeführt sind und über eine spezielle Vorrichtung ein Unterdruck im Flüssigkeitsbehälter erzeugt wird, sodaß bei Ausfall des Ventilsystems keine unmittelbare Gefahr für den Patienten droht. Folgt man der Dimensionierung der dort angegebenen Konstruktion, kommt man auf das zitierte Problem: Der letztlich vom Volumen begrenzte Flüssigkeitsbehälter kann nur mit niedrigkonzentriertem Insulin betrieben werden, woraus eine hohe Nachfüllrate (ca. alle 2 Wochen) resultiert, die sicherlich aus der Sicht des Patienten nicht akzeptabel ist.

Bei einer Vorrichtung gemäß der US-PS 4209014 wird ein elastischer, aber permeabler Kunststoff mit Hilfe eines elektromagnetisch betriebenen Kolbens mechanisch ausgepreßt. Kunststoffe mit nicht geschlossenen Strukturen, wie beispielsweise Schaumgummi oder Polyurethanschaum, sind für Pharmaka geringgradig durchlässig. Wird solch ein Kunststoff zusätzlich mit Hilfe eines Kolbens mechanisch komprimiert, so kann die Durchflußrate beträchtlich erhöht werden. Dem Vorteil der einfachen Konstruktion stehen jedoch beträchtliche Nachteile gegenüber. So ist - vergleiche die zitierte Patentschrift - die bei Kompression erreichte Menge letztlich doch nur um einen Faktor 3 zu erhöhen. Der bei nicht komprimiertem Kunststoff gegebene Durchsatz benötigt wiederum einen Primärdruck im Flüssigkeitsgefäß. Darüber hinaus ist jedoch die Langzeitbeständigkeit einer solchen Pumpe mit Sicherheit nicht gegeben, da für eine exakte Dosierung die Dauerelastizität eines solchen Kunststoffes bekannterweise nicht gegeben ist. Auch der Energieverbrauch bei der Kompression dieses elastischen Materials ist laut zitierter Patentschrift erheblich (größer 1 Watt).

Durch die AT-PS 378123 des Patentinhabers wurde eine Pumpe bekannt, die über einen Elektromagnet angesteuert wird. Durch eine spezielle Konfiguration von zwei Kolben und zwei Zylindern aus Metall wird die Langzeitbeständigkeit erhöht. Diese Pumpe arbeitet nach dem Schöpfprinzip und benötigt keine konventionellen Ventile mit Ventilfedern. Besonderes Merkmal dieser Konstruktion ist dabei das geringe Schöpfvolumen (0,3 bis 1,5 μl pro Zyklus). Das Pumpvolumen wird dabei durch die Anzahl der Pumpzyklen definiert.

Durch die AT-PS 360636, die im wesentlichen eine Pumpe der eingangs erwähnten Art betrifft, wurde ein wurstförmiger Schlauch aus einem speziellen Kunststoffmaterial bekannt, der sich bei spezieller Konstruktion mit einer Flüssigkeit so aufblasen läßt, daß bei Entleerung in einem bestimmten Bereich der Elastizität ein annähernd konstanter Druckverlauf (Angabe des Erfinders ca. 10 %) entstehen soll. Nachteilig dabei ist jedoch das benötigte hohe Residialvolumen, so daß die Pumpe unrationell arbeitet. Die bekannte Konstruktion ist ausschließlich für externe Anwendung konzipiert. Es können nur wenige Kunststoffe verwendet werden, da nur wenige Kunststoffe die notwendigen Charakteristika aufweisen, wobei es fraglich ist, ob die verwendbaren Kunststoffe soweit biokompatibel sind, daß auch sensible Medikamente, wie beispielsweise Insulin, verwendet werden können. Da weiters das Material nicht vorgepannt ist, kann nur der lineare Bereich der Kraft-Dehnungskurve genutzt werden, was zu einem weiteren Verlust an Volumen führt. Ein konstanter Druck wird in der zitierten Patentschrift nicht geoffenbart.

Extern betriebene Pumpen arbeiten heute nach denselben Prinzipien wie implantierbare (Kolbenpumpen, Rollenquetschen...), darüberhinaus werden häufig miniaturisierte, mechanisch über Spindelgetriebe betriebene Spritzvorrichtungen verwendet. Solche Pumpen sind grundsätzlich auch in der Geschwindigkeit regelbar. Zum Stand der Technik gehören auch Programme zur Steuerung, beispielsweise eines vorkalkulierten Tagesprofils für Insulin (W.J.Spencer, "A Review of Programmed Insulin Delivery Systems" in IEEE Transactions on Biomedical Engineering, Vol.BME-28 No.3, März 81).

Heute verwendete externe Pumpen weisen Nachteile, wie zu großes Volumen des gesamten Pumpsystems und daher schon aus sozialen Gründen nur begrenzte Akzeptanz durch den Patienten, zu großer Bedienungsaufwand, insbesondere bei programmierbaren Pumpen, mit dadurch bedingt häufigen Bedienungsfehlern durch den Patienten (Risiko einer Hypo-bzw. Hyperglykämie) auf, weshalb solche Pumpen für betagte Patienten nicht verwendbar sind. Weiters kommt es zu Problemen der Hygiene (Duschen, Baden) beim Tragen der Pumpen und zu Problemen im Bereich der Ausgangskanülen (Injektionsnadel), und dadurch bedingter Entzündungsgefahr bei längerem Liegen, Sensibilität, Gefahr der Dislokation bei Zug am Kathether. Einen weiteren Nachteil stellt der hohe Preis, im Bereich zwischen 30000,-- und 50000,--österr. Schilling dar.

Bei treibgasbetriebenen Pumpen kommt es zu Veränderung der Dosis bei Temperaturschwankungen (Fieber). Infolge des geringen absoluten Druckes des Treibgases entstehen von der Meereshöhe abhängige Änderungen des Pumpvolumens und damit Probleme beim Schifahren, Fliegen....usw.

Aufgrund schädigender Wirkungen des Treibgases muß ein Metallfaltenbalg verwendet werden, was einen weiteren Nachteil darstellt. Weiters tritt bei treibgasbetriebenen Pumpen eine Veränderung der Pumprate bis zu 50 % bei Entleerung der Pumpe aufgrund der Eigencharakteristik des zur Trennung von Flüssigkeit und Treibgas notwendigen Metallfaltenbalges auf.

Aufgabe der Erfindung ist es, unter Vermeidung der Nachteile der bekannten Vorrichtungen, eine Pumpe (Basalratenpumpe) zu schaffen, welche die folgenden Qualitätskriterien erfüllt:

Einfache Konstruktion, geringstes Volumen und Gewicht, geringste Herstellungskosten, verbunden mit der Möglich-keit, die Pumpe als Einmalartikel zu verwenden, geringsten Bedienungsaufwand, höchste Bedienungssicherheit, daher auch Verwendbarkeit für betagte Patienten, kein bzw. vernachlässigbares Residualvolumen, Herstellbarkeit aus insulinkompatiblem Material und präzise Funktion über den gesamten Bereich der Entleerung.

Erreicht wird dieses Ziel bei einer Vorrichtung der eingangs erwähnten Art, wenn gemäß der Erfindung das Reservoir ringförmig um den Grundteil angeordnet und an diesem abgestützt ist und wenn das Reservoir von einem radial elastisch vorgespannten ringförmigen Körper umfaßt ist, der das flüssige Arzneimittel im Reservoir auch während der Entleerung unter Druck hält.

Die vorliegende Erfindung erfüllt die im letzten Abschnitt beschriebenen Kriterien. Die Unterscheidung zu bisherigen Ideen liegt in der speziellen Geometrie der Anordnung zur Erzielung eines konstanten Druckes während des gesamten Entleervorganges. Definitionsgemäß ist die Volumskonstanz dann erreicht, wenn der auf das Reservoir wirkende Druck während des gesamten Entleervorganges konstant bleibt. Basis der Erfindung ist folgende physikalische Überlegung:

Der Druck ist als Quotient zwischen Kraft und Fläche, auf welche die Kraft wirkt, definiert. Da nun die Geometrie des Reservoirs bei der Entleerung Änderungen unterliegt, kann Druckkonstanz während des gesamten Entleervorganges nur dann erreicht werden, wenn

1. die Verringerung des Volumens und damit der Oberfläche des Reservoirs (Delta F) während der Entleerung, eine proportionale Verringerung der Kraft (Delta P) nach sich zieht,

2. ein lineares Verhältnis zwischen Kraft und Oberfläche besteht und

3. die Möglichkeit besteht, die Konstruktion der vorgegebenen Kennlinie des elastischen Ringes anzupassen bzw. umgekehrt.

Aufgrund der erfindungsgemäßen Konstruktion kann bei Entleerung der Pumpe (Verringerung des Volumens) der Umfang des zylindrischen Ringes proportional reduziert werden. Die Reduktion des Umfanges (auch proportional der Reduktion des Radius) führt unter Verwendung der Charakteristika federnder Elemente zu einer proportionalen Entspannung des Federelementes und damit zu einer proportionalen Verringerung der Kraft. Da bei gleichbleibender Höhe des Zylinders, der Druck durch den Quotient zwischen Kraft und Fläche (Umfang der zylindrischen Konstruktion) definiert ist, ist der Druck dann konstant, wenn ein proportionaler Zusammenhang zwischen Dehnung und Kraft besteht. Erfindungsgemäß kann der lineare Zusammenhang in der Gleichung zwischen Dehnung und Kraft ($\sigma = k\epsilon + d$) dann in einen proportionalen Zusammenhang ($\sigma = k\epsilon$) umgewandelt werden, wenn durch konstruktive Maßnahmen eine Verschiebung der Koordinaten $\sigma, \epsilon$ möglich ist. In der vorliegenden Erfindung ist dies durch geeignete vorspannung des federnden Ringes sowie durch geeignete Wahl des Radius des Grundteiles erreichbar.

Metallische Federn und auch Kunststoffe besitzen eine Spannungs/Dehnungskurve, die über einen Bereich einen linearen Zusammenhang von Spannung und Dehnung aufweist. Zur Realisierung der zitierten Bedingungen ist es auch notwendig, die axialen Kräfte zu beseitigen, was durch besondere Maßnahmen im Rahmen der Erfindung geschieht.

Abgesehen vom grundsätzlich neuen geometrischen Prinzip zur Erzielung einer druckkonstanten Entleerung, ergibt die vorliegende Erfindung weitere besondere Vorteile. Das Reservoir kann in einen einzigen Silikonkautschukring integriert werden, welcher aufgrund seiner Vorspannung auch die notwendige Kraft auf die Flüssigkeit ausübt. Diese Integration des Reservoirs in den Ring ermöglicht eine extrem kostengünstige und reproduzierbare Herstellung durch übliche Spritzgußverfahren. Durch balgartige Einbuchtungen bzw. Einschnitte des Silikonkautschukringes im Bereich des Reservoirs werden axiale Kräfte, die zu einer Nichtlinearität führen würden, verhindert.

Ein besonderer Vorteil für externe Anwendung ist durch die Möglichkeit der Trennung zwischen Applikationsund Pumpteil gegeben. Durch diese Trennung läßt sich nicht nur auf sehr einfache Art und Weise, nämlich durch Eindrücken des Applikationsteiles in den Pumpenteil, die Pumpe in Betrieb nehmen, sondern es kann darüberhinaus durch Applikationsteile, die mit verschieden langen Reduktionskanülen versehen sind, auch das gewünschte Pumpvolumen pro Zeit variiert werden. In Kenntnis der Tatsache, daß insulinabhängige Patienten pro Tag zwischen IO und IOO Insulineinheiten benötigen, bietet gerade diese Moglichkeit der Einstellung der Pumprate einen besonderen Vorteil. Nachdem auch alle weiteren Teile aus Kunststoffen bestehen, die im Spritzgußverfahren ökonomisch hergestellt werden können, ist die Forderung nach geringem Preis optimal erfüllt. Zur Erhöhung des Langzeitverhaltens - beispielsweise bei einer implantierbaren Ausführungsform zur Aufbringung der Kraft - kann auch eine zylindrische Metallfeder vorgesehen und vorzugsweise in den Silikonkautschukring eingebettet werden. Neben Silikonkautschuk können insbesondere in der implantierbaren Ausführung auch eine Reihe anderer biokompatibler und bioresistenter Materialien Verwendung finden, wie z.B. Titan für das Gehäuse und Polyurethan für den Flüssigkeitsbehälter. Auch kann neben der zylindrisch kreisrunden jede andere Geometrie verwendet werden, die zur Realisierung des zitierten physikalischen Prinzips dient (z.B. Vielecke).

Die Erfindung wird an Hand der Zeichnungen näher erläutert. Es zeigt,

Fig. I den Zusammenhang zwischen Spannung (N/mm²) und Dehnung (ε) eines Kunststoffes (Silikonkautschuk),

Fig. 2 einen Achsschnitt durch ein Ausführungsbeispiel einer erfindungsgemäß ausgebildeten Vorrichtung, wobei in der linken Hälfte der Zustand bei voll befülltem Reservoir und in der rechten Hälfte der Zustand knapp vor der Entleerung veranschaulicht ist,

Fig. 3 eine Draufsicht auf die Vorrichtung der Fig. 2 bei abgenommenem Deckel, und

Fig. 4 eine Ansicht des Deckels von unten,

Fig. 5 einen Achsschnitt durch eine erfindungsgemäße Vorrichtung in einer implantierbaren Variante,

Fig. 6 ein Schaubild für den Zusammenhang von abgegebenem Volumen und Zeit, und

Fig. 7 ein Schaubild analog Fig. 6, jedoch bei einer Temperatur des Mediums, die gegenüber der Temperatur des Mediums, das der Aufzeichnung der Fig. 6 zugrundegelegen hat, geändert wurde.

Aus dem Spannungs/Dehnungsdiagramm der Fig. I ist ersichtlich, daß es ausgehend von einer maximalen Dehnung im Bereich von 400 %, bei Entlastung zunächst zu einem stark nicht linearen Zusammenhang zwischen Spannung und Dehnung kommt, dem ein linearer Bereich folgt, der zwischen 300 und I00 % Dehnung liegt. Die weitere Entspannung des elastischen Materials verläuft anschließend daran nicht linear. Der Anstieg des linearen Teiles ist mit "k", die σ-Koordinate des Schnittpunktes mit der σ-Achse mit "d" bezeichnet.

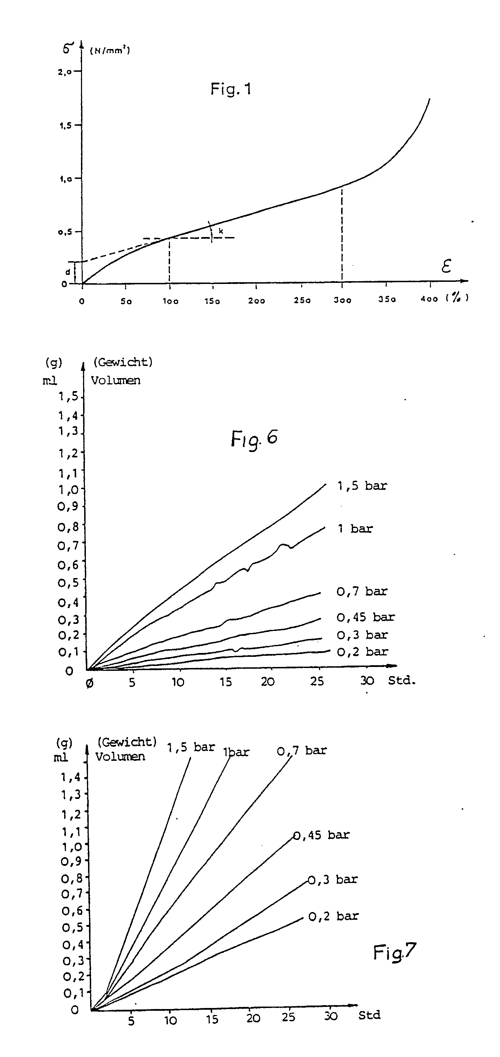

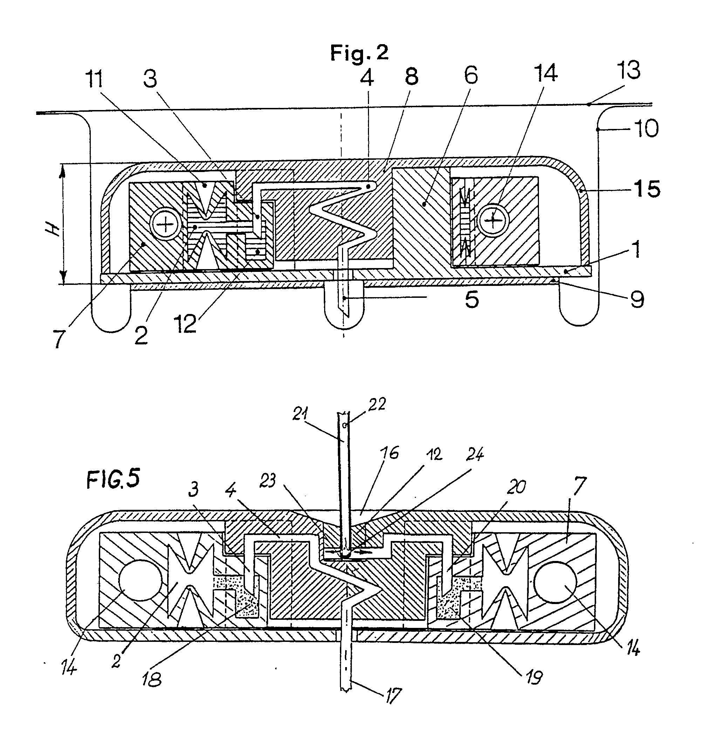

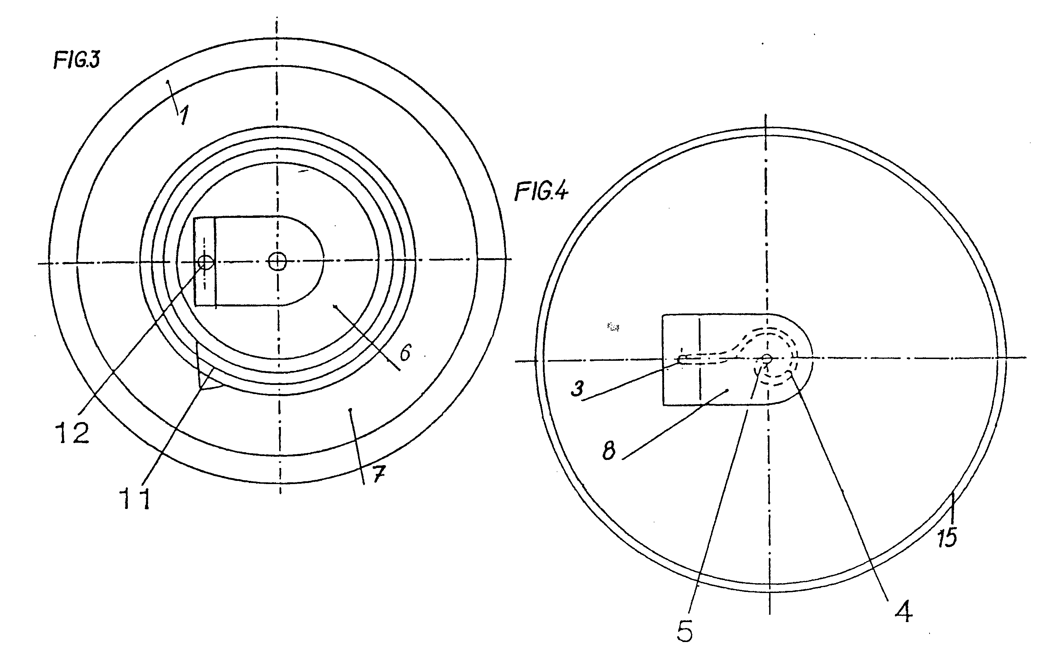

Aus den Fig. 2 und 3 ist der Gehäuseunterteil 1 ersichtlich, der mit einem zylinderförmigen Grundteil 6 verbunden ist, um welchen das flexible Reservoir 2 für das Arzneimittel ringförmig angeordnet ist. Ein um das Reservoir 2 angeordneter, radial elastisch vorgespannter ringförmiger Körper 7 setzt die Flüssigkeit im Reservoir 2 unter Druck, wobei die Kompressionskräfte vom Grundteil 6 aufgenommen werden. Vorteilhaft kann das Reservoir 2 in den ringförmigen Körper 7 integriert werden, der beispielsweise aus Silikonkautschuk gefertigt ist. Dies erlaubt die kostengünstige Herstellung im Spritzgußverfahren. Zur Vermeidung axialer Kräfte, die die Linearität des Druckes während des Entleervorganges beeinflussen würden, können balgförmige Einschnitte bzw. Einbuchtungen II am

Reservoir 2 ausgebildet werden. Der Applikationsteil 8, welcher auch den der gewünschten Fließgeschwindigkeit angepaßten Ausströmkanal 4 beinhaltet, wird zu Betriebsbeginn in den zylindrischen Grundteil 6 eingeschoben, wobei eine Verdrehsicherung dafür sorgt, daß das Anstechen mit der Anstechnadel 3 zwecks Verbindung des Kanals 4 mit dem Reservoir 2 präzise erfolgen kann. Das Ende des Ausströmkanals 4 ist mit einer Ausgangskanüle, vorzugsweise einer Injektionsnadel 5, verbunden. Der zylindrische Applikationsteil 8 wird vorteilhaft mit dem Gehäuseoberteil I5 in einem Stück gefertigt. Zur langzeit-luftdichten Verpackung, insbesondere bei Medikamenten, die gasdicht verpackt werden sollen, wird die Vorrichtung mittels einer zweiteiligen Metallfolie I0 und I3,vorzugsweise aus Aluminium, verpackt. Zur Anbringung der Pumpe am Körper des Patienten dient eine doppelseitig klebende Folie 9, welche am Gehäuseunterteil 1 angebracht ist. Die Folie 9 dient auch zur Positionierung der Pumpe in der Verpackung I0, I3. Zur Verbesserung des Zeitverhaltens - manche elastische Materialien unterliegen einer Alterung während der Lagerung des Gerätes -kann um das Reservoir 2 eine radial vorgespannte zylindrische Metallfeder I4 (z.B. eine torusartig ausgebildete Schraubenfeder) angebracht werden, welche auch im elastisch vorgespannten ringförmigen Körper 7 eingegossen werden kann. Zum Füllen des Reservoirs 2 mit dem gewünschten Arzneimittel dient ein Füllventil I2, welches vorzugsweise aus einem vorgespannten zylindrischen Silikonkautschukteil besteht. In einer implantierbaren Version-(Fig.5)der Vorrichtung wird das Arzneimittel über das Füllventil I2 percutan nachgefüllt, die Vorrichtung enthält weiters ein in Strömungsrichtung vor dem Ausströmkanal 4 geschaltetes Filter sowie einen an die Ausgangskanüle 5 angeschlossenen Katheter.

Erfindungsgemäße Kriterien der Pumpe betreffen neben der speziellen Geometrie der Anordnung, die Lösbarkeit des Applikationsteiles 8 vom Gehäuseunterteil I zum Zwecke der Lagerung und der Inbetriebnahme der Pumpe durch einfaches Zusammendrücken. Weiters ergibt sich durch die Lösbarkeit des Applikationsteiles vom Gehäuseunterteil I die Möglichkeit der Veränderung des Pumpvolumens/Zeit, durch Austausch des Applikationsteiles 8. Die Applikation der Pumpe direkt am Körper über eine doppelseitige Klebefolie 9, die bei Entnahme aus der Verpackung automatisch zur Verfügung steht, sowie insbesondere die Integration zwischen Reservoir 2 und dem vorgespannten Ring 7, welcher auch über die Metallfeder I4 verstärkt werden kann, sind weitere erfindungsgemäße Merkmale. Ein weiteres wesentliches Kriterium betrifft die balgförmigen Ausschnitte bzw. Einbuchtungen II am Reservoir 2 zur Vermeidung axialer Kräfte. Die Dosiervorrichtung 4 (Ausströmkanal) kann entweder aus einem dünnen Metallrohr bestehen, erfindungsgemäß kann die Reduktion jedoch auch durch Ätzen von Metallschichten über hochpräzise lithographische Vorlagen oder auch durch Filter realisiert werden. Die Inbetriebnahme der druckkonstanten Pumpe ist extrem einfach. Zunächst wird die obere Metallfolie 13 der Verpackung von der unteren Metallfolie 10 gelöst, dann der Applikationsteil 8 in den Pumpenteil gedrückt, hernach die Pumpe aus der weichen Verpackung entnommen, wodurch die Klebeseite der Klebefolie 9 frei wird und die Pumpe daher mittels der Klebefolie direkt an den Körper des Patienten geklebt werden kann. Die Größe der Pumpe entspricht etwa jener heute üblicher EKG-Elektroden. Die Technologie biokompatibler Klebefolien ist aus diesem Bereich bekannt.

Die Pumpe ist auch implantierbar, dabei sind bekannte Merkmale zu verwenden. Die Pumpe ist wie üblich permanent in Betrieb. Die Anstechvorrichtung 12 muß dabei perkutan leicht erreichbar sein, wozu eine trichterförmige Einbuchtung 16 am Applikationsteil 8 vorgesehen wird, die durch die Bauchdecke getastet werden kann. Das Nachfüllventil 12 besteht bevorzugt aus Silikonkautschuk Die Ausgangskanüle 5 wird über ein Verbindungsstück 17 mit einem (nicht dargestellten) Kathether verbunden und vor der Dosiervorrichtung 4 (Ausströmkanal) wird vorteilhaft ein Filter 18 positioniert. Ebenso kann ein Filter 19 vor dem Einlaß in das Reservoir 2 im Bereich der Einmündung des Nachfüllkanals 20 angeordnet werden. Beim Einstechen der Nachfüllnadel 21, die mit einer Zulauföffnung 22 versehen ist, ist zur Begrenzung der Eindringtiefe der Nadel 22 in den Applikationsteil 8, in diesem ein Metallplättchen 23 angeordnet, an welchem die Nachfüllnadel 21 mit ihrem bevorzugt geschlossenen Vorderende zur Anlage kommt; die Nadel 22 ist mit seitlichen Austrittsöffnungen 24 für das nachzufüllende Medikament versehen. Bei der implantierbaren Ausführung ist das Reservoir 2 mit dem Dosierteil (Reduktionskanüle 4) permanent verbunden.

Bei externer Applikation können die Abmessungen der Vorrichtung entsprechend klein sein. Fig. 2 ist dabei (bis auf die Reduktionskanüle 4) maßstabsgetreu. Das Maß H beträgt in der Wirklichkeit 12 mm.Das Volumen des Reservoirs 2 beträgt je nach Medikament zwischen 1 und 5 ml. In der dargestellten Ausführung ist die Länge der Reduktionskanüle 4 vierzig mm, der äußere Durchmesser 0,2 mm und der innere Durchmesser 0,02 mm. Damit läßt sich bei entsprechender Mischung des Medikamentes mit Glyzerin die Tagesmenge (25 - 30 Stunden) erreichen.

Aus den Schaubildern der Fig. 6 und 7 sind die Abgabemengen in der Zeit ersichtlich. Fig. 6 gibt dabei das Ergebnis von Durchflußmessungen an standardisierten Reduktionskanülen 4 an, deren Innendurchmesser 60 μ und deren Länge 40 mm betragen hat. Das Durchflußmedium enthielt 89 % steriles, pyrogenfreies Glycerin. Die Temperatur hat 22°C betragen, mit einer Toleranz von ± 1°C. Erfolgt die Volumsmessung über Gewichtsmessung mit Hilfe einer Mikrogrammwaage, so ist dabei die auftretende Verdunstung im Endergebnis einzurechnen. Fig. 7 gibt das Schaubild einer Durchflußmessung analog zu Fig. 6, jedoch bei einer Umgebungstemperatur von 35°C bei einer Toleranz von ± 1°C.

Standardwert für die Abgabemenge ist 1 ml pro Tag. Die Dauer der Abgabe richtet sich demgemäß nach dem Gesamtvolumen. In der externen Ausführung ist es im Zusammenhang mit der Lebensweise der Patienten sinnvoll, die Pumpe einmal pro Tag zu wechseln.

In der implantierbaren Version sind die Dimensionen etwa zu verdoppeln, da wesentlich mehr Füllvolumen notwendig ist. Bei einer Erhöhung der Dimensionen um 100 % läßt sich ein Füllvolumen erreichen, welches für einen etwa 4-wöchigen Betrieb ausreicht.

Die Dimensionen der Injektionsnadel bei der subkutanen Applikation über die externe Vorrichtung betragen 5 mm Länge und 0,3 mm Außendurchmesser.

**Ansprüche**

1. Vorrichtung zur strömungskonstanten Abgabe flüssiger Arzneimittel, bestehend aus einem Gehäuse mit einem Unterteil (1), einem im Gehäuse untergebrachten, komprimierbaren flexiblen Reservoir (2) zur Aufnahme des Arzneimittels, gegebenenfalls einem Füllventil (12) zur Füllung des Reservoirs (2), einem mit Reservoir (2) verbundenen bzw. verbindbaren Ausströmkanal (4) definierten Querschnitts und definierter Länge, sowie einer zur Applikation geeigneten Ausgangskanüle (5), wobei mit dem Gehäuse-Unterteil (1) ein zylinderförmiger Grundteil (6) verbunden ist, um welchen Grundteil (6) herum, das flexible Reservoir (2) für das Arzneimittel angeordnet ist, dadurch gekennzeichnet, daß das Reservoir ringförmig um den Grundteil (6) angeordnet und an diesem abgestützt ist und daß das Reservoir (2) von einem radial elastisch vorgespannten ringförmigen Körper (7) umfaßt ist, der das flüssige Arzneimittel im Reservoir (2) auch während der Entleerung unter Druck hält.

2. Vorrichtung nach Anspruch 1, dadurch gekennzeichnet, daß das Reservoir (2) mit dem ringförmigen Körper (7) zu einem einzigen, vorzugsweise aus Silikonkautschuk gefertigten ringförmigen Teil, integriert ist.

3. Vorrichtung nach Anspruch I und 2, dadurch gekennzeichnet, daß das Reservoir zur Vermeidung der Wirkung axialer Kräfte axial verlaufende balgförmige Einbuchtungen (II) aufweist.

4. Vorrichtung nach Anspruch I bis 3, gekennzeichnet durch einen separaten, in eine Ausnehmung des zylindrischen Grundteiles einschiebbaren und gegen Verdrehung gesicherten zylindrischen Applikationsteil (8), welcher den der gewünschten Fließgeschwindigkeit angepaßten Ausströmkanal (4) beinhaltet, dessen erstes Ende mit einer Anstechnadel (3) zwecks Verbindung mit dem Reservoir (2) und dessen anderes Ende mit einer Ausgangskanüle, vorzugsweise einer Injektionsnadel (5) verbunden ist, wobei weiters der zylindrische Applikationsteil (8) mit dem Gehäuse-Oberteil (I5) verbunden sein kann.

5. Vorrichtung nach Anspruch 1 bis 4, dadurch gekennzeichnet, daß ihr eine zweiteilige Metallfolie (IO, I3), vorzugsweise aus Aluminium, zur langzeit-luftdichten Verpackung zugeordnet ist.

6. Vorrichtung nach Anspruch 5, gekennzeichnet durch eine zwischen der Unterseite des Gehäuse-Unterteils (I) und der Metallfolie (IO) positionierte doppelseitig klebende Folie (9).

7. Vorrichtung nach Anspruch 2 bis 6, dadurch gekennzeichnet, daß zur Erhöhung der Langzeitstabilität eine um das Reservoir (2) angeordnete radial vorgepannte zylindrische Metallfeder (I4) vorgesehen ist.

8. Vorrichtung nach Anspruch 7, dadurch gekennzeichnet, daß die Metallfeder (I4) im ringförmigen Körper untergebracht ist.

9. Implantierbare Vorrichtung nach Anspruch I, 2, 3, 5, 7 und 8, dadurch gekennzeichnet, daß ein mit dem Reservoir (2) in Verbindung stehendes Füllventil (I2) zur perkutanen Nachfüllung, gegebenenfalls ein in Strömungsrichtung dem Ausströmkanal (4) vorgeschaltetes Filter, sowie vorzugsweise ein an die Ausgangskanüle (5) angeschlossener Kathether vorgesehen sind.

## Claims

1. Device for constant-flow delivery of liquid medicaments, consisting of a housing with a lower part (1), a compressible flexible reservoir (2) accommodated in the housing for the purpose of receiving the medicament, optionally a fill-up valve (12) for filling the reservoir (2), an outflow channel (4) of defined cross-section and defined length which is connected or connectable to the reservoir (2), and an outlet cannula (5) appropriate for the administration, a cylindrical base part (6) being connected to the lower part (1) of the housing, around which base part (6) the flexible reservoir (2) for the medicament is arranged, characterised in that the reservoir is arranged annularly around the base part (6) and is supported on the latter, and in that the reservoir (2) is enclosed by a radially elastically prestressed annular body (7), which keeps the liquid medicament in the reservoir (2) under pressure even during discharging.

2. Device according to Claim 1, characterised in that the reservoir (2) is integrated with the annular body (7) to form a single annular part preferably made from silicone rubber.

3. Device according to Claim 1 and 2, characterised in that, in order to avoid the effect of axial forces, the reservoir has axially extending bellows-like indents (II).

4. Device according to Claim 1 to 3, characterised by a separate cylindrical application part (8) which can be pushed into a recess of the cylindrical base part, is secured against torsion and contains the outflow channel (4) which is adapted to the desired flow rate and whose first end is connected to a puncturing needle (3) for the purpose of connection to the reservoir (2) and whose other end is connected to an outlet cannula, preferably an injection needle (5), it being possible moreover for the cylindrical application part (8) to be connected to the upper part (15) of the housing.

5. Device according to Claim 1 to 4, characterised in that it is allocated a two-part metal foil (10, 13), preferably of aluminium, for long-term airtight packing.

6. Device according to Claim 5, characterised by a two-sided adhesive foil (9) positioned between the underside of the lower part (1) of the housing and the metal foil (10).

7. Device according to Claim 2 to 6, characterised in that, in order to increase the long-term stability, a radially prestressed cylindrical metal spring (14) is provided around the reservoir (2).

8. Device according to Claim 7, characterised in that the metal spring (14) is accommodated in the annular body.

9. Implantable device according to Claim 1, 2, 3, 5, 7 and 8, characterised in that a fill-up valve (12) in communication with the reservoir (2) for percutaneous refilling, if appropriate a filter arranged upstream of the outflow channel (4) in the direction of flow, and preferably a cathether connected to the outlet cannula (5) are provided.

**Revendications**

1. Dispositif pour délivrer un médicament liquide à débit constant, composé d'un carter comprenant une partie inférieure (1), un réservoir flexible susceptible d'être comprimé (2) logé dans le carter, pour recevoir le médicament, le cas échéant une soupape de remplissage (12) pour remplir le réservoir (2), un canal d'évacuation (4), relié ou susceptible d'être relié au réservoir (2), dont la section transversale et la longueur sont définies, ainsi qu'une canule de sortie (5) appropriée à l'application, une partie de base de forme cylindrique (6) reliée à la partie inférieure de carter (1), autour de laquelle est disposé le réservoir flexible (2) pour le médicament, caractérisé en ce que le réservoir est disposé de façon annulaire autour de la partie de base (6) et prend appui sur celle-ci, le réservoir (2) étant entouré par un corps annulaire (7) radialement élastique et précontraint, qui maintient sous pression le médicament liquide dans le réservoir (2), même lorsqu'il se vide.

2. Dispositif selon la revendication 1, caractérisé en ce que le réservoir (2) avec le corps annulaire (7) sont intégrés pour former une partie annulaire unique, fabriquée de préférence en caoutchouc au silicone.

3. Dispositif selon les revendications 1 et 2, caractérisé en ce que le réservoir présente des empreintes (11) en forme de soufflet, s'étendant axialement, pour éviter l'action d'efforts axiaux.

4. Dispositif selon les revendications 1 à 3, caractérisé par une partie d'application cylindrique (8), insérable dans un évidement de la partie de base cylindrique et bloquée en rotation, qui contient le canal d'évacuation (4) adapté à la vitesse de liquide souhaitée, dont la première extrémité est reliée à une aiguille de percement (3), pour assurer la liaison avec le réservoir (2), et dont l'autre extrémité est reliée à une canule de sortie, de préférence une aiguille d'injection (5), la partie d'application cylindrique (8) pouvant être en plus reliée à la partie supérieure de carter (15).

5. Dispositif selon les revendications 1 à 4, caractérisé en ce qu'il lui est associé une feuille métallique en deux parties (10,13), de préférence en aluminium, pour assurer un emballage étanche à l'air pendant une longue durée.

6. Dispositif selon la revendication 5, caractérisé par une feuille à double face adhésive (9), positionnée entre la face inférieure de la partie inférieure de carter (1) et la feuille métallique (10).

7. Dispositif selon les revendications 2 à 6, caractérisé en ce qu'un ressort métallique cylindrique (14) précontraint, disposé radialement autour du réservoir (2), est prévu pour augmenter la stabilité dans le temps.

8. Dispositif selon la revendication 7, caractérisé en ce que le ressort métallique (14) est logé dans le corps annulaire.

9. Dispositif implantable selon les revendications 1,2,3,5,7 et 8, caractérisé en ce que sont prévus une soupape de remplissage (12), en liaison avec le réservoir (2), pour assurer un remplissage percutané ultérieur, le cas échéant un filtre placé en circuit en amont du canal d'évacuation (4), dans le sens d'écoulement, ainsi que de préférence un cathéter raccordé à la canule de sortie (5).

Fig. 1

Fig. 6

Fig.7

**Fig. 2**

**FIG. 5**

FIG.3

FIG.4

EP 0 242 351 B1